(19) Europäisches Patentamt
European Patent Office
Office européen des brevets

(11) Veröffentlichungsnummer: **0 238 445**
**A1**

# (12) EUROPÄISCHE PATENTANMELDUNG

(21) Anmeldenummer: **87810149.2**

(22) Anmeldetag: **16.03.87**

(51) Int. Cl.[4]: **C 07 D 307/81**
A 01 N 47/10

(30) Priorität: **20.03.86 CH 1124/86**

(43) Veröffentlichungstag der Anmeldung:
**23.09.87 Patentblatt 87/39**

(84) Benannte Vertragsstaaten:
**BE CH DE ES FR GB GR IT LI NL**

(71) Anmelder: **CIBA-GEIGY AG**
**Klybeckstrasse 141**
**CH-4002 Basel (CH)**

(72) Erfinder: **Ehrenfreund, Josef, Dr.**
**Amselstrasse 11**
**CH-4123 Allschwil (CH)**

## (54) Substituierte Hydrazincarbonsäureester.

(57) Neue gegebenenfalls substituierte 2-Fluorcarbonyl-2-(2,3-dihydrobenzofuran-7-yl)-hydrazincarbonsäureester der Formel

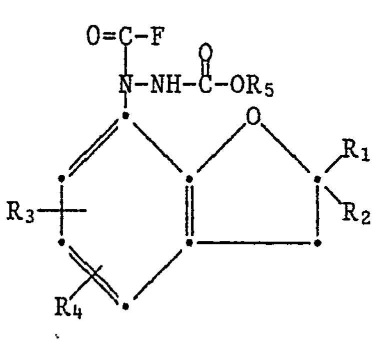

worin
$R_1$ und $R_2$ unabhängig voneinander Wasserstoff oder $C_1$-$C_3$-Alkyl;
$R_3$ und $R_4$ unabhängig voneinander Wasserstoff, Halogen, $C_1$-$C_4$-Alkyl oder $C_1$-$C_4$-Alkoxy; und
$R_5$ $C_1$-$C_4$-Alkyl bedeuten;

Verfahren zur Herstellung dieser Verbindungen sowie diese enthaltende Mittel zur Verwendung in der Schädlingsbekämpfung, insbesondere zur Bekämpfung von Pflanzen und Tiere befallenden Insekten. Die neuen Verbindungen weisen besonders hohe Wirksamkeit gegen pflanzenschädigende saugende Insekten auf.

EP 0 238 445 A1

**Beschreibung**

Substituierte Hydrazincarbonsäureester

Die vorliegende Erfindung betrifft neue, gegebenenfalls substituierte 2-Fluorcarbonyl-2-(2,3-dihydrobenzofuran-7-yl)-hydrazincarbonsäureester, Verfahren zu ihrer Herstellung und ihre Verwendung in der Schädlingsbekämpfung.

Die neuen Verbindungen gemäss der vorliegenden Erfindung haben die Formel I

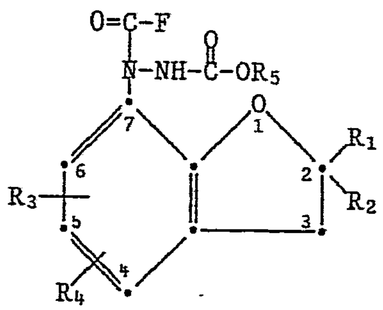

(I),

worin

$R_1$ und $R_2$ unabhängig voneinander Wasserstoff oder $C_1$-$C_3$-Alkyl;
$R_3$ und $R_4$ unabhängig voneinander Wasserstoff, Halogen, $C_1$-$C_4$-Alkyl oder $C_1$-$C_4$-Alkoxy; und
$R_5$ $C_1$-$C_4$-Alkyl bedeuten.

Von besonderem Interesse sind erfindungsgemäss Verbindungen der Formel I, worin $R_1$, $R_2$, $R_3$ und $R_4$ unabhängig voneinander Wasserstoff oder Methyl und $R_5$ Methyl oder Aethyl, vorzugsweise Methyl, bedeuten.

Bevorzugt sind daneben wegen ihrer biologischen Bedeutung solche Verbindungen der Formel I, worin $R_1$, $R_2$ und $R_5$ Methyl bedeuten.

Unter Halogen bei $R_3$ une $R_4$ ist Fluor, Chlor, Brom oder Jod zu verstehen, insbesondere Fluor oder Chlor.

Die Alkyl- und Alkoxygruppen bei $R_1$ bis $R_5$ können geradkettig oder verzweigt sein. Beispiele solcher Gruppen sind u.a.: Methyl, Methoxy, Aethyl, Aethoxy, Propyl, Propoxy, Isopropyl, Isopropoxy, n-Butyl, n-Butoxy, iso-Butyl usw.

Die Verbindungen der Formel I können hergestellt werden, indem man

a) eine Verbindung der Formel II

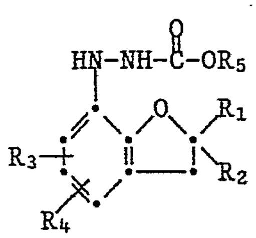

(II)

mit Fluorphosgen ($COF_2$) oder Monofluorphosgen (COClF) oder

b) eine Verbindung der Formel III

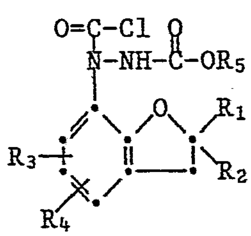

(III)

mit einer den Austausch von Chlor gegen Fluor bewirkenden Verbindung umsetzt, wobei in den Formeln II und III die Reste $R_1$ bis $R_5$ die vorstehend angegebenen Bedeutungen besitzen.

Das zu den erfindungsgemässen Verbindungen der Formel I führende Verfahren a) entspricht der bereits bekannten Umsetzung substituierter Hydrazincarbonsäureester mit Phosgen unter Bildung von entsprechenden 2-Chlorcarbonyl-Derivaten (vgl. europäische Patentschrift 0 067 471, Japanische Patentschriften J5 9222 490, J5 8222 085 und J5 8222 083). Fluorphosgenisierungen sind im Prinzip aus J.Chem.Soc. 1948, 2183, bekannt.

Das Verfahren wird bei einer Reaktionstemperatur zwischen -10° und 120° C, meist zwischen 20° und 80° C, bei normalem oder erhöhtem Druck und vorzugsweise in einem inerten Lösungs- oder Verdünnungsmittel durchgeführt. Als Lösungs- oder Verdünnungsmittel eignen sich z.B. Aether und ätherartige Verbindungen, wie Diäthyläther, Dipropyläther, Dioxan, Dimethoxyäthan und Tetrahydrofuran; aliphatische, aromatische sowie halogenierte Kohlenwasserstoffe, insbesondere Benzol, Toluol, Xylole, Chloroform und Chlorbenzol; sowie Ester, z.B. Essigsäureäthylester.

Beim Verfahren b) werden Chlorcarbonylverbindungen der Formel III durch Umsetzung z.B. mit Alkalifluoriden in geeigneten Lösungsmitteln. gegebenenfalls unter Zusatz von Kronenverbindungen oder Phasentransferkatalysatoren, oder aber durch Umsetzung mit wasserfreier Flussäure, Antimonfluorid oder andern geeigneten Reagentien, die einen Austausch von Chlor durch Fluor bewirken, (vgl. Angew. Chemie 89 797; 1977), in die entsprechenden erfindungsgemässen Verbindungen der Formel I übergeführt.

Die als Ausgangsstoffe verwendeten substituierten Hydrazincarbonsäureester der Formel II und 2-Chlorcarbonyl-hydrazincarbonsäureester der Formel III sind bekannt bzw. können analog bekannten Verfahren erhalten werden. So kann man die Hydrazincarbonsäureester der Formel II erhalten durch Umsetzung von gegebenenfalls substituierten 2,3-Dihydrobenzofuran-7-yl-hydrazinen mit einem entsprechenden Chlorkohlensäureester (vgl. europäische Patentanmeldung 0 048 040). Die 2-Chlorcarbonyl-Hydrazincarbonsäureester der Formel III sind gemäss dem unter Verfahren a) vorstehend angegebenen Schrifttum zugänglich.

Pestizid, insbesondere insektizid, wirksame 2-(2,3-Dihydrobenzofuran-7-yl)-Hydrazincarbonsäureester sind bereits aus der US-Patentschrift 4.467.104, der europäischen Patentschrift 0 067 471 und der europäischen Patentanmeldung 0 048 040 bekannt. Gegenüber diesen bekannten Verbindungen unterscheiden sich die erfindungsgemässen Wirkstoffe der Formel I strukturell durch das Vorliegen einer 2-Fluorcarbonylhydrazin-Gruppierung.

Es wurde nunmehr überraschenderweise gefunden, dass die vorliegenden Verbindungen der Formel I bei guter Pflanzenverträglichkeit und geringer Warmblütertoxizität überlegene Wirksamkeit als Schädlingsbekämpfungsmittel aufweisen. Sie eignen sich vor allem zur Bekämpfung von Pflanzen und Tieren befallenden Schädlingen, speziell zur Vertilgung saugender Insekten.

Insbesondere eignen sich die Verbindungen der Formel I zur Bekämpfung von Insekten der Ordnungen Lepidoptera, Coleoptera, Homoptera, Heteroptera, Diptera, Thysanoptera, Orthoptera, Anoplura, Siphonaptera, Mallophaga, Thysanura, Isoptera, Psocoptera und Hymenoptera sowie von Vertretern der Ordnung Akarina.

Die gute pestizide Wirkung der erfindungsgemässen Verbindungen der Formel I entspricht einer Abtötungsrate (Mortalität) von mindestens 50-60 % der erwähnten Schädlinge.

Mit Hilfe der erfindungsgemässen Verbindungen der Formel I können vor allen pflanzenschädigende Insekten, speziell pflanzenschädigende Insekten in Zier- und Nutzpflanzungen, insbesondere in Baumwollkulturen, Gemüsekulturen, Reiskulturen und Obstkulturen bekämpft werden. In diesem Zusammenhang ist hervorzuheben, dass die genannten Verbindungen sich sowohl durch eine stark ausgeprägte systemische als auch Kontakt-Wirkung gegen saugende Insekten, vor allem gegen Insekten der Familie Aphididae (wie z.B. Aphis fabae, Aphis craccivora und Myzus persicae), welche sich mit herkömmlichen Mitteln nur schwierig bekämpfen lassen, auszeichnen.

Die Verbindungen der Formel I zeichnen sich weiterhin durch eine gute Wirkung gegen larvale Insektenstadien und Nymphen, speziell von saugenden Schadinsekten, aus. Insbesondere können die Verbindungen der Formel I mit ausgezeichnetem Erfolg gegen pflanzenschädigende Zikaden, speziell in Reis-Kulturen, eingesetzt werden.

Die Verbindungen der Formel I eignen sich ferner zur Bekämpfung von Ektoparasiten, z.B. Lucilia sericata, sowie von Zecken an Haus-und Nutztieren, z.B. durch Tier-, Stall- und Weidebehandlung.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide, chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen, Vernebeln, Verstäuben, Verstreuen oder Giessen, werden ebenso wie die Mittel den angestrebten Zielen und den gegebenen Verhältnissen entsprechend gewählt.

Die Formulierung, d.h. die den Wirkstoff der Formel I, bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen

Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden Wirkstoffes der Formel I oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäure-methyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder - sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläther gruppen enthaltenden Polyäthylenoxidaddukte an Polypropylenglykol, Aethylendiaminopolypropylenglykol und Alkylpolpropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxidaddukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben:
"Mc Cutcheon's Detergents and Emulsifiers Annual"
MC Publishing Corp., Ridgewood, New Jersey, 1979;
Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, eines Wirkstoffes der Formel I oder Kombinationen davon mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Beispiel 1: Herstellung von 2-(2-Fluorcarbonyl)-2-(2,3-dihydro-2,2-dimethylbenzofuran-7-yl)-hydrazincarbonsäuremethylester (Verbindung Nr. 1)

5 g 2-(2,3-Dihydro-2,2-dimethylbenzofuran-7-yl)-hydrazincarbonsäuremethylester werden in 50 ml Toluol dispergiert und auf dieses in einem Autoklaven befindliche Gemisch werden 10 g Fluorphosgen aufgepresst. Die Mischung wird im Autoklaven 24 Stunden bei Raumtemperatur gerührt und anschliessend wird das überschüssige Gas mittels Luft ausgeblasen. Die Reaktionslösung wird anschliessend dreimal mit Wasser und einmal mit 5%iger Natriumbicarbonatlösung gewaschen, über Natriumsulfat getrocknet und das Lösungsmittel in vacuo entfernt. Der kristalline Rückstand wird aus Tetrachlormethan umkristallisiert.

Die so erhaltene Verbindung Nr. 1 der Formel

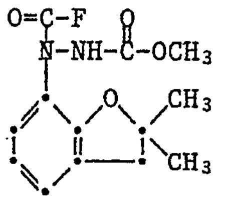

besitzt einen Schmelzpunkt von 104-106° C.

Wie vorstehend beschrieben sind die folgenden Verbindungen der Formel I erhältlich:

| Verb. Nr. | $R_1$ | $R_2$ | $R_3$ | $R_4$ | $R_5$ | Smp. C° |
|---|---|---|---|---|---|---|
| 1 | $-CH_3$ | $-CH_3$ | H | H | $-CH_3$ | 104-106° |
| 2 | $-CH_3$ | $-CH_3$ | 5-$CH_3$ | H | $-CH_3$ | 107-110° |
| 3 | $-CH_3$ | $-CH_3$ | H | 4-$CH_3$ | $-CH_3$ | 121-123° |
| 4 | $-CH_3$ | $-CH_3$ | H | H | $-C_2H_5$ | 101,5-102,5° |
| 5 | $-CH_3$ | $-CH_3$ | H | 4-$CH_3$ | $-C_2H_5$ | 95-98° |
| 6 | $-CH_3$ | $-CH_3$ | H | H | $-C_3H_7$(n) | |
| 7 | $-CH_3$ | $-CH_3$ | H | H | $-C_3H_7$(i) | |
| 8 | $-CH_3$ | $-CH_3$ | 5-$CH_3$ | H | $-C_2H_5$ | |
| 9 | $-CH_3$ | $-CH_3$ | H | 4-$CH_3$ | $-C_3H_7$(i) | |
| 10 | $-CH_3$ | $-CH_3$ | 5-$CH_3$ | 4-$CH_3$ | $-CH_3$ | |

Beispiel 2:

Formulierungen für Wirkstoffe der Formel I gemäss Beispiel 1 resp. Kombinationen dieser Wirkstoffe mit andern Insektiziden oder Akariziden (% = Gewichtsprozent):

| 1. Spritzpulver | a) | b) | c) |
|---|---|---|---|
| Wirkstoff oder Wirkstoffkombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

2. Emulsions-Konzentrat

Wirkstoff oder Wirkstoffkombination 10 %
Octylphenolpolyäthylenglykoläther (4-5 Mol AeO) 3 %
Ca-Dodecylbenzolsulfonat 3 %
Ricinusölpolyglykoläther (36 Mol AeO) 4 %
Cyclohexanon 30 %
Xylolgemisch 50 %

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

4. Extruder-Granulat

Wirkstoff oder Wirkstoffkombination 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

5. Umhüllungs-Granulat

Wirkstoff oder Wirkstoffkombination 3 %
Polyäthylenglykol (MG 200) 3 %
Kaolin 94 %

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

Wirkstoff oder Wirkstoffkombination 40 %
Aethylenglykol 10 %
Nonylphenolpolyäthylenglykoläther (15 Mol AeO) 6 %
Na-Ligninsulfonat 10 %

Carboxymethylcellulose 1 %
37%ige wässrige Formaldehyd-Lösung 0,2 %
Silikonöl in Form einer 75%igen wässrigen Emulsion 0,8 %
Wasser 32 %

Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

Beispiel 3: Wirkung gegen Musca domestica

Je 50 g frisch zubereitetes Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 400 ppm ergibt. Nach dem Durchmischen des Substrates lässt man das Aceton mindestens 20 Stunden lang verdampfen.

Dann werden pro Wirkstoff je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansatz ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss Beispiel 1 zeigen gute Wirkung im obigen Test.

Beispiel 4: Wirkung gegen Lucilia sericata (Larven)

Zu 9 ml eines Zuchtmediums wird bei 50°C I ml einer 0,5 % Aktivsubstanz enthaltenden wässrigen Zubereitung gegeben. Nun werden ca. 30 frisch geschlüpfte Lucilia sericata-Larven zum Zuchtmedium gegeben. Nach 48 und 96 Stunden wird die insektizide Wirkung durch Ermittlung der Abtötungsrate festgestellt.

Verbindungen der Formel I gemäss Beispiel 1 zeigen in diesem Test gute Wirkung gegen Lucilia sericata.

Beispiel 5: Wirkung gegen Aedes aegypti (Larven)

Auf die Oberfläche von 150 ml Wasser, das sich in einem Behälter befindet, wird so viel einer 0,1%igen acetonischen Lösung des Wirkstoffes pipettiert, dass eine Konzentration von 400 ppm erhalten wird. Nach Verdunsten des Acetons wird der Behälter mit 30 bis 40 2-tägigen Aedes-Larven beschickt. Nach 2 und 7 Tagen wird die Mortalität geprüft.

Verbindungen der Formel I gemäss Beispiel 1 zeigen gute Wirkung im obigen Test.

Beispiel 6: Kontaktwirkung auf Aphis craccivora

In Töpfen angezogene 4-5 Tage alte Erbsenkeimlinge (Vicia faba) werden vor Versuchsbeginn mit je ca. 200 Individuen der Spezies Aphis craccivora besiedelt. Die so infestierten Pflanzen werden 24 Stunden später mit einer wässrigen Zubereitung der zu prüfenden Verbindung bis zur Tropfnässe direkt besprüht. Man verwendet pro Test-Verbindung zwei Pflanzen. Eine Auswertung der erzielten Abtötungsrate erfolgt nach weiteren 24 und 72 Stunden. Der Versuch wird bei 21-22°C und einer relativen Luftfeuchtigkeit von etwa 55 % durchgeführt.

Verbindung Nr. 1 gemäss Beispiel 1 ergibt bei 1 ppm eine Mortalität von 80-100 % in diesem Test.

Beispiel 7: Systemische Wirkung auf Aphis craccivora

Bewurzelte Bohnenpflanzen werden in Töpfe, welche 600 ccm Erde enthalten, verpflanzt. Anschliessend giesst man 50 ml einer Zubereitung der zu prüfenden Verbindungen (erhalten aus einem 25%igen Spritzpulver) in einer Konzentration von 400 ppm direkt auf die Erde in den Töpfen.

Nach 24 Stunden werden auf die oberirdischen Pflanzenteile Blattläuse der Spezier Aphis craccivora gesetzt, und über die Pflanzen wird ein Plastikzylinder gestülpt, um die Läuse vor einer eventuellen direkten Kontakt- oder Gaswirkung der Testsubstanz zu schützen.

Die Auswertung der erzielten Abtötung erfolgt 48 und 72 Stunden nach Versuchsbeginn. Pro Testsubstanz werden zwei Pflanzen, je eine in einem separaten Topf, verwendet. Der Versuch wird bei 25°C und ca. 70 % relativer Luftfeuchtigkeit durchgeführt.

Verbindungen der Formel I gemäss Beispiel 1 zeigen gute Wirkung in diesem Test.

Beispiel 8: Kontaktwirkung auf Myzus persicae

Etwa 4 bis 5 Tage alte, in Wasser (Becher von 200 ml Inhalt) angezogene Erbsenkeimlinge (Vicia faba) werden vor Versuchsbeginn jeweils mit ca. 200 Individuen der Spezies Myzus persicae (OP-resistenter Stamm) besiedelt. Die so behandelten Pflanzen werden 24 Stunden später mit einer wässrigen Suspension enthaltend 25 und 50 ppm der zu prüfenden Verbindung bis zur Tropfnässe direkt besprüht. Man verwendet pro Testsubstanz zwei Pflanzen. Eine Auswertung der erzielten Abtötungsrate erfolgt 24 Stunden nach Wirkstoff-Applikation. Der Versuch wird bei 21-22°C und etwa 55 % relativer Luftfeuchtigkeit durchgeführt.

Die Verbindung Nr. 1 gemäss Beispiel 1 ergibt eine 80-100%ige Mortalität in diesem Test bei den angegebenen Wirkstoff-Konzentrationen.

Beispiel 9: Systemische Wirkung auf Myzus persicae

Bewurzelte Kohlpflanzen im 4- bis 5-Blatt-Stadium werden in Töpfe, welche 60 ccm Erde enthalten, verpflanzt. Ansschliessend werden 50 ml einer wässrigen Formulierung der zu prüfenden Verbindung (erhalten aus einem 25%igen Spritzpulver) jeweils in einer Konzentration von 400 ppm direkt auf die Erde aufgegossen.

Nach 24 Stunden werden auf die oberirdischen Pflanzenteile der behandelten Pflanzen Blattläuse der Spezies Myzus persicae gesetzt und die Pflanzen mit Plastikzylindern überdeckt, um die Blattläuse vor einer eventuellen direkten Kontakt- oder Gaswirkung der Testsubstanz zu schützen.

Die Auswertung der erzielten %-Abtötung erfolgt 48 Stunden nach Versuchsbeginn. Pro Testsubstanz werden zwei Pflanzen, je eine in separaten Töpfen, verwendet. Der Versuch wird bei ca. 25°C und 60 % relativer Luftfeuchtigkeit durchgeführt.

Verbindungen der Formel I gemäss Beispiel 1 zeigen gute Wirkung in obigem Test.

Beispiel 10: Blattpenetrations-Wirkung auf Aphis craccivora

In etwa 8 cm hohe Kunststoffbecher (Durchmesser etwa 6 cm) wird ein passender kleiner Zweig von Vicia faba gelegt, der mit Blattläusen der Spezies Aphis craccivora stark infiziert ist. Der Becher wird mit einem Kunststoffdeckel, der in der Mitte eine ausgestanzte Oeffnung von 2 cm Durchmesser aufweist, bedeckt. Auf die in dem Deckel befindliche Oeffnung wird ein Blatt einer Vicia faba-Pflanze gelegt, ohne dieses Blatt von der eingetopften Pflanze abzutrennen.

Das Blatt wird dann mit einem zweiten Lochdeckel auf dem Becher über der Oeffnung des ersten Deckels fixiert. Von der Unterseite her, d.h. durch die Oeffnung des ersten Deckels hindurch, infizieren nun die in dem Becher befindlichen Blattläuse das obenliegende Blatt der Futterpflanze. Auf der Oberseite wird auf das Blatt eine wässrige Zubereitung des zu prüfenden Wirkstoffes in einer Konzentration von 400 ppm mittels eines Pinsels gleichmässig aufgetragen. Es wird geprüft, ob die einseitig auf die Oberseite des Blattes der Futterpflanze aufgetragene Testsubstanz in genügender Menge durch das Blatt hindurch auf dessen Unterseite diffundiert, um dort saugende Blattläuse abzutöten.

Der Versuch wird bei etwa 20°C und 60 % relativer Luftfeuchtigkeit durchgeführt. Die Auswertung auf %-Mortalität erfolgt 48 Stunden nach Wirkstoffapplikation.

Verbindungen der Formel I gemäss Beispiel 1 zeigen gute Wirkung in obigem Test.

Beispiel II: Frassgift-Wirkung auf Laodelphax striatellus und Nilaparvata lugens (Nymphen);

Der Test wird an wachsenden Pflanzen durchgeführt. Dazu werden jeweils 4 Reispflanzen (Dicke des Stengels 8 mm) mit einer Höhe von ca. 20 cm in Töpfe (Durchmesser von 8 cm) eingepflanzt.

Die Pflanzen werden auf einem Drehteller mit 100 ml einer acetonischen Lösung enthaltend 800 ppm des jeweiligen Wirkstoffes besprüht. Nach dem Antrocknen des Spritzbelages erfolgt die Besiedlung jeder Pflanze mit je 20 Nymphen der Testtiere im dritten Stadium. Um die Zikaden am Entweichen zu hindern, wird über die besiedelten Pflanzen jeweils ein beidseitig offener Glaszylinder gestülpt und dieser mit einem Gaze-Deckel abgedeckt. Die Nymphen werden bis zum Erreichen des folgenden Entwicklungsstadiums über 10 Tage an der behandelten Pflanze gehalten. Die Auswertung auf %-Mortalität erfolgt 1, 4 und 8 Tage nach der Behandlung.

Verbindungen der Formel I gemäss Beispiel 1 zeigen gute Wirkung in diesem Test.

Beispiel 12: Insektizide Frassgift-Wirkung

Ca. 25 cm hohe eingetopfte Baumwollpflanzen werden mit wässrigen Wirkstoffemulsionen besprüht, die den Wirkstoff in einer Konzentration von 400 ppm enthalten.

Nach dem Antrocknen des Sprühbelages werden die Baumwollpflanzen mit Spodoptera littoralis- bzw. Heliothis virescens-Larven im dritten larvalen Stadium besiedelt. Der Versuch wird bei 24°C und etwa 60 % relativer Luftfeuchtigkeit durchgeführt. Nach 120 Stunden wird die %-Mortalität der Test-Insekten gegenüber unbehandelten Kontrollen bestimmt.

Die Verbindung Nr. 1 gemäss Beispiel 1 zeigt 80-100 % Wirkung (Mortalität) gegen Spodoptera-Larven in diesem Test.

Beispiel 13: Ovizide Wirkung auf Heliothis virescens

Entsprechende Mengenanteile einer benetzbaren pulverförmigen Formulierung, enthalten 25 Gew.-% des zu prüfenden Wirkstoffes, werden mit jeweils soviel Wasser vermischt, dass sich wässrige Emulsionen mit einer Wirkstoffkonzentration von 800 ppm ergeben.

In diese wirkstoffhaltigen Emulsionen werden eintägige Eigelege von Heliothis auf Cellophan® während drei Minuten eingetaucht und dann auf Rundfiltern abgenutscht. Die so behandelten Gelege werden in Petrischalen ausgelegt und in der Dunkelheit aufbewahrt. Nach 6 bis 8 Tagen wird die Schlupfrate im Vergleich zu unbehandelten Kontrollen festgestellt. Zur weiteren Auswertung wird die zur 80-100%igen Abtötung der Eier erforderliche minimale Wirkstoffkonzentration bestimmt.

Verbindungen der Formel I gemäss Beispiel 1 zeigen gute Wirkung in obigem Test.

Beispiel 14: Wirkung gegen Bodeninsekten (Diabrotica balteata)

Es werden 350 ml Erde (bestehend aus 95 Vol.-% Sand und 5 Vol.-% Torf) mit jeweils 150 ml von wässrigen Emulsionszubereitungen vermischt, die den zu prüfenden Wirkstoff in einer Konzentration von 400 ppm enthalten. Dann werden Plastikbecher, die einer oberen Durchmesser von ca. 10 cm haben, mit der so behandelten Erde teilweise gefüllt. Pro Becher werden zehn Larven von Diabrotica balteata im dritten Larvalstadium eingesetzt, vier Maiskeimlinge eingepflanzt und die Becher mit Erde aufgefüllt. Die gefüllten Becher werden mit Plastikfolie abgedeckt und bei einer Temperatur von etwa 22° C gehalten. Zehn Tage nach dem Ansatz wird die in den Bechern enthaltene Erde abgesiebt und die zurückbleibenden Larven hinsichtlich ihrer Mortalität geprüft.

Verbindungen der Formel I gemäss Beispiel 1 zeigen gute Wirkung im obigem Test.

Beispiel 15: Systemische insektizide Wirkunggegen Nilaparvata lugens

Reispflanzen werden mit 5 ml einer Versuchslösung enthaltend 400 ppm der zu prüfenden Verbindung angegossen und nach 1, 2 resp. 3 Wochen mit jeweils 20 Nilaparvata-Nymphen besiedelt. Die Auswertung der erzielten Abtötungsrate erfolgt jeweils 6 Tage nach der Besiedlung.

Verbindungen der Formel I gemäss Beispiel 1 zeigen gute Wirkung in diesem Test.

Beispiel 16: Wirkung gegen Nephotettix cincticeps (Nymphen)

Der Test wird an wachsenden Pflanzen durchgeführt. Dazu werden ca. 20 Tage alte Reispflanzen mit einer Höhe von etwa 15 cm in Töpfe (Durchmesser 5,5 cm) eingepflanzt.

Die Pflanzen werden auf einem Drehteller mit jeweils 100 ml einer acetonischen Lösung enthaltend 400 ppm des zu prüfenden Wirkstoffs besprüht. Nach dem Antrocknen des Spritzbelages erfolgt die Besiedlung jeder Pflanze mit je 20 Nymphen der Testtiere im zweiten oder dritten Stadium. Um die Zikaden am Entweichen zu hindern, wird über die besiedelten Pflanzen jeweils ein Plexiglasszylinder gestülpt und dieser mit einem Gaze-Deckel abgedeckt. Die Nymphen werden für 5 Tage an der behandelten Pflanze, die mindestens 1 mal nachgegossen werden muss, gehalten. Der Versuch wird bei einer Temperatur von ca. 23° C, bei 55 % relativer Luftfeuchtigkeit und mit einer Belichtungsperiode von 16 Stunden durchgeführt.

Die erfindungsgemässen Verbindungen Nr. 1 und 3 gemäss Beispiel 1 zeigen in diesem Test gute Wirkung.

Beispiel 17: Wirkung gegen Aonidiella aurantii

Es wird eine Lösung des Wirkstoffes mit einer Konzentration von 100 ppm in einem Aceton-Wasser-Gemisch (1:1) hergestellt. In diese Lösung werden für kurze Zeit Kartoffelknollen eingetaucht, die 14 Tage zuvor mit einer Population von Aonidielle aurantii (rote Citrus-Schildlaus) infiziert worden waren. Nach dem Trocknen der so behandelten infizierten Kartoffelknollen werden diese während 6 bis 8 Wochen bei 22-24° C in einer mit einem Siebdeckel abgedeckten Schale deponiert. Nach dieser Zeit wird die % Mortalität, die allfällige Entwicklung beweglicher Jungtiere und die Oviposition kontrolliert und ausgewertet.

Die Verbindungen Nr.1 und 3 gemäss Beispiel 1 zeigen gute Wirkung in diesem Test.

**Patentansprüche**

1. Verbindung der Formel I

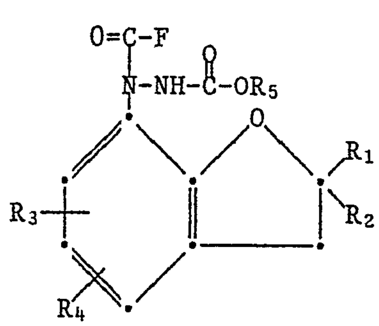

worin

$R_1$ und $R_2$ unabhängig voneinander Wasserstoff oder $C_1$-$C_3$-Alkyl;

$R_3$ und $R_4$ unabhängig voneinander Wasserstoff, Halogen, $C_1$-$C_4$-Alkyl oder $C_1$-$C_4$-Alkoxy; und

$R_5$ $C_1$-$C_4$-Alkyl bedeuten

2. Verbindungen der Formel I gemäss Anspruch 1, dadurch gekennzeichnet, dass $R_1$, $R_2$, $R_3$ und $R_4$ unabhängig voneinander Wasserstoff oder Methyl und $R_5$ Methyl oder Aethyl bedeuten.

3. Verbindung der Formel I gemäss Anspruch 2, dadurch gekennzeichnet, dass $R_5$ Methyl bedeutet.

4. Verbindung der Formel I gemäss Anspruch 2, dadurch gekennzeichnet, dass $R_1$, $R_2$ und $R_5$ Methyl

bedeuten.

5. Verbindung gemäss Anspruch 3 der Formel

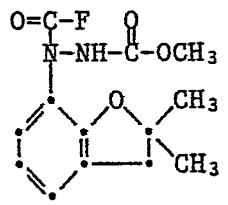 .

6. Verbindung gemäss Anspruch 3 der Formel

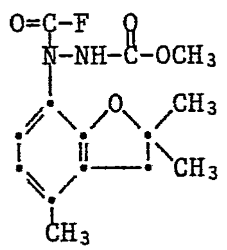 .

7. Verfahren zur Herstellung einer Verbindung der Formel I gemäss einem der Ansprüche 1 bis 6, dadurch gekennzeichnet, dass man

a) eine Verbindung der Formel II

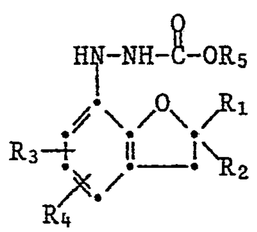 (II)

mit Fluorphosgen oder Monofluorphosgen oder

b) eine Verbindung der Formel III

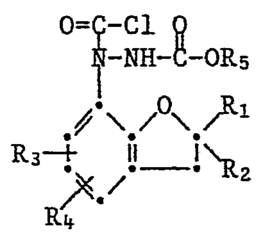 (III)

mit einer den Austausch von Chlor gegen Fluor bewirkenden Verbindung umsetzt, wobei in den Formeln II und III die Reste $R_1$ bis $R_5$ die in Anspruch 1 angegebenen Bedeutungen haben.

8. Schädlingsbekämpfungsmittel, welches als aktive Komponente eine Verbindung gemäss einem der Ansprüche 1 bis 6 zusammen mit geeigneten Trägern und/oder anderen Zuschlagstoffen enthält.

9. Verwendung einer Verbindung gemäss einem der Ansprüche 1 bis 6 zur Bekämpfung von Insekten und Vertretern der Ordnung Akarina an Tieren und Pflanzen.

10. Verwendung gemäss Anspruch 9 zur Bekämpfung von pflanzenschädigenden Insekten.

11. Verwendung gemäss Anspruch 10 zur Bekämpfung von pflanzenschädigenden saugenden Insekten.

12. Verfahren zur Bekämpfung von Insekten und Vertretern der Ordnung Akarina, dadurch gekennzeichnet, dass man die Schädlinge bzw. deren verschiedene Entwicklungsstadien und/oder ihren Aufenthaltsort mit einer pestizid wirksamen Menge einer Verbindung der Formel I gemäss einem der

Ansprüche 1 bis 6 oder mit einem Mittel enthaltend neben Zusatz- und Trägerstoffen eine pestizid wirksame Menge dieser Verbindung, in Kontakt bringt oder behandelt.

Europäisches Patentamt

# EUROPÄISCHER RECHERCHENBERICHT

Nummer der Anmeldung

EP 87810149.2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X,D | EP - A1 - 0 048 040 (SHELL)<br>* Formula III; Zusammenfassung *<br>& US-A-4 467 104<br>-- | 1,6,8-12 | C 07 D 307/81<br>A 01 N 47/10 |
| A | US - A - 4 302 592 (TIEMAN)<br>* Formula (6); Beispiel 1 *<br>-- | 1 | |
| A | US - A - 4 550 121 (PILGRAM)<br>* Ansprüche 1,4 *<br>---- | 1,8 | |

RECHERCHIERTE SACHGEBIETE (Int. Cl.4)

C 07 D 307/00

Der vorliegende Recherchenbericht wurde für alle Patentansprüche erstellt.

| Recherchenort | Abschlußdatum der Recherche | Prüfer |
|---|---|---|
| WIEN | 22-05-1987 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument